# EUROPEAN PATENT SPECIFICATION

(11) **EP 1 519 832 B1**
(45) Date of publication and mention of the grant of the patent: **20.10.2010**
(21) Application number: 03739253.7
(22) Date of filing: 19.06.2003
(51) Int. Cl.: B32B 5/04, B32B 5/08, A61F 13/15

(54) **STRAND-REINFORCED ELASTOMERIC COMPOSITE LAMINATES, GARMENTS INCLUDING THE LAMINATES, AND METHODS OF MAKING THE LAMINATES**
STRANG-VERSTÄRKTE ELASTOMERE LAMINATE, KLEIDUNGSSTÜCKE DIESE BEINHALTEND UND VERFAHREN ZUR HERSTELLUNG DER LAMINATE
STRATIFIES COMPOSITES ELASTOMERES RENFORCES PAR DES FILS, VETEMENTS COMPRENANT CES STRATIFIES, ET PROCEDES DE FABRICATION DE CES STRATIFIES

(30) Priority: 02.07.2002 US 187761; 26.12.2002 US 329990; 26.12.2002 US 330042
(43) Date of publication of application: 06.04.2005
(73) Proprietor: KIMBERLY-CLARK WORLDWIDE, INC., Neenah, WI 54956 (US)
(72) Inventor: NECULESCU, Cristian, M., Neenah, WI 54956 (US); ZHOU, Peiguang, Appleton, WI 54915 (US); GARRETT, JR., Lance, J., Neenah, WI 54956 (US); WIDEMAN, Daniel, J., Menasha, WI 54952 (US); ACHTER, Bruce, Neenah, WI 54956 (US)
(74) Representative: Davies, Christopher Robert
(86) International application number: PCT/US2003/019659
(87) International publication number: WO 2004/005018

(56) References cited:
- EP-A- 0 330 716
- EP-A- 0 802 251
- WO-A-80/00676
- WO-A-02/085624
- US-A1- 2002 081 423

## Description

This invention is directed to elastic composite materials including elastomeric adhesive film reinforced with elastic strands, and methods of making such strand-reinforced elastomeric composite laminates. This invention is also directed to garments having a strand-reinforced elastomeric adhesive film laminate adjacent one or more openings of the garment.

Personal care garments often include elasticized portions to create a gasket-like fit around certain openings, such as waist openings and leg openings. Laminates made from conventional elastic strands and elastic attachment adhesive are often used to create such elasticized portions. However, such laminates can be rough and uncomfortable.

Furthermore, such laminates may cause red-marking on a wearer's skin if the fit is too tight and may result in leakage from the garment if the fit is too loose.

One type of elastomeric laminate is a stretch-bonded laminate that includes elastic strands produced from an extruder and bonded to a facing sheet or sheets using a hot melt adhesive. Laminates including pre-made elastic strands can be processed online but require an elastic attachment adhesive with high add-on in order to reduce strand slippage. The cost of making stretch-bonded laminates can be relatively high due to the cost of the facing sheet or sheets, plus the cost of the elastic strands, plus the cost of the adhesive.

Another type of elastomeric laminate can be made using a vertical filament laminate-stretch-bonded laminate (VFL-SBL) process. However, the VFL-SBL process must be in off-line operation due to process complexity.

Layers having filament elastics are disclosed in EP-A-0330716, US-A-2002/0081423, WO-A-80/00676, EP-A-0802251 and also W)-A-02/085624, this latter document being published after the earliest priority date of the present application and thus falls within the terms of Article 54(3) EPC.

One drawback associated with conventional elastic strands and elastic laminates around garment openings are leakage and fit problems, particularly around leg openings when the garment is loaded. Such problems result from weakening tension or uneven tension distribution of the leg elastics. More specifically, elastic tension is weakened during wearing because of strand relaxation and strand slippage at elevated temperatures. To prevent leakage, tension of the leg elastics can be increased by increasing the number of strands or by using a high denier of strands at a higher stretching ratio. However, an increase in leg elastic tension is likely to cause a "red mark" on a wearer's skin because tension of the garment is actually concentrated on the narrow surface of the strand instead of the whole elastic laminate. Plus, the tension on the conventional elastic laminate can not be transported or distributed uniformly during use especially when legs are moving. Also, current elastic strand-based laminates cannot be die-cut to curve and fit the body exactly. Making curved elastic strand laminates in a high-speed assembly process requires very complex and precise control, which is associated with large capital investment.

Elastomeric adhesive compositions are multifunctional in the sense that they function as an elastomer in a nonwoven composite while also serving as a hot melt adhesive for bonding substrates. Elastomeric adhesive compositions in the form of elastomeric adhesive films are currently recognized as suitable for use in the manufacture of personal care articles. More particularly, elastomeric adhesive compositions can be used to bond facing materials, such as spunbond, to one another while simultaneously elasticizing the resulting laminate. The resulting laminate can be used to form an elastomeric portion of an absorbent article, such as a region surrounding a waist opening and/or a leg opening.

Non-woven elastic adhesive film laminates may require high output of adhesive add-on to achieve a tension target for product application. High add-on of the film laminate may generate a bulky, thick feel and appearance, and high cost. Furthermore, the high adhesive output requirement for the film formation would make on-line processing even more difficult due to the limitation of hot melt equipment output capacity. Also, such film lamination processes are relatively complex and need more precise control than strand lamination since a film edge thinning effect may cause the film to break during stretching.

Some elastomeric adhesive compositions lose their adhesiveness when the compositions are stretched and then bonded between two nonwoven substrates. The elasticity of these elastomeric adhesive compositions (in terms of tension decay) is negatively affected when laminates including the compositions are aged at elevated temperatures, for example around 54 ° C (130 degrees Fahrenheit), which is commonly experienced under hot boxcar storage conditions. It appears that the poor tension and adhesion of such elastomeric adhesive compositions results from the chosen base polymer, tackifier, and plasticizer chemistries as well as the unbalanced ratio of polymer to low molecular weight species in the formulation.

There is a need or desire for an elastomeric laminate that can be used to create elasticized portions of a personal care garment, wherein the laminate does not display high tension decay or delamination. There is a further need or desire for a personal care garment including elasticized portions that possess a soft feel and comfortable fit, while providing adjustable tension to minimize leakage. There is yet a further need or desire for a method of making strand-reinforced elastic composites and laminates with improved processability in which film breakage, strand breakage, and strand alignment problems are minimized or avoided.

### SUMMARY OF THE INVENTION

In response to the discussed difficulties and problems encountered in the prior art, a new strand-reinforced elastomeric adhesive film laminate, and a garment including a strand-reinforced elastomeric adhesive film laminate, and a method of making strand-reinforced elastomeric composites and laminates have been discovered.

The composites and laminates of the invention have superior elastic and adhesion properties. The method of making these composites and laminates provides improved processability and minimization or avoidance of film breakage, strand breakage, and strand alignment problems.

The elastic composite laminates of the invention are made up of a combination of extruded reinforcing strands and elastomeric adhesive film. The strands may be adhered to, and possibly even partially or wholly embedded in, the elastomeric adhesive film. A layer of spunbond or other facing material can be laminated along one or both surfaces of the film to provide the elastic composite laminates of the invention. The combination of reinforcing strands and the elastomeric adhesive film significantly and advantageously reduces the rate and extent of tension decay, as well as improving adhesion properties of the spunbond laminates compared to spunbond laminates including elastomeric adhesive film without reinforcing strands. Additionally, the tension on the elastomeric composite laminate is distributed evenly across the width of the laminate instead of being concentrated only on the strands, thus reducing or eliminating red marking and providing a better fit. Furthermore, the reinforcing strands enable the composite tension to be tunable while preserving the soft feel and aesthetic properties of the laminate. Tuning can be achieved by adjusting the output of the strand or film add-on, adjusting the stretching ratio, selection of substrates, as well as polymer formulas. Another benefit of using the elastomeric composite laminate rather than conventional elastics is that the laminate can be die-cut into any shape for better fit and comfort without losing tension.

Garments of the invention include one or more openings for a body part such as, for example, a neck opening, a wrist opening, an arm opening, a waist opening, a leg opening, and/or an ankle opening. The garment includes a substrate that defines the opening or openings, with an elastomeric composite laminate attached to the substrate adjacent the opening or openings. The elastomeric composite laminate is used in place of conventional elastic strands or elastic laminates to provide better fit and comfort, less leakage and more tension control for disposable garment applications. One surface of the laminate can be attached to a substrate of the garment adjacent the opening or openings while the other surface of the laminate may include a layer of spunbond or other facing material. Alternatively, facing materials can be laminated along both surfaces of the film prior to attaching the laminate to the substrate.

The garment of the invention may be a personal care garment, medical garment, industrial workwear garment, or the like. In one embodiment, the garment is a pant-like garment with a chassis including a substrate. The chassis defines a waist opening and two leg openings. The elastomeric composite laminate is attached to the substrate adjacent each of the leg openings.

The composites and laminates can be made by casting an elastomeric adhesive film onto a chill roll. The film may be passed onto a second chill roll. In any case, a plurality of elastic strands is extruded onto the film while the film is on a chill roll to create a strand-reinforced composite. The strand-reinforced composite is then stretched, suitably by at least 200%. The stretched composite can be laminated between two facing sheets to form a strand-reinforced composite laminate.

Stretching the film and strand components as a single unit affords the added benefits of a more robust manufacturing method. The ability to control processability is especially critical for converting line operations where start/stop situations need to be minimized for economic reasons. Furthermore, by extruding the elastic strands directly onto the film, the strands are self-aligning on the film in the machine direction without moving in the cross direction or breaking. While the film reinforces the strands, the strands also provide reinforcement to the film, especially along the edges of the film. The elastic composite made from the mutually reinforced strand and film combination is much stronger and resistant to breaking than elastomeric adhesive film alone or elastic strands alone. Additionally, the composite made according to the method of the invention can be stretched even higher and the web speed can be run even faster than strand-reinforced elastomeric composites made by separately stretching the film and the strands, with a lesser chance of film or strand breakage or strand alignment problems.

The method of the invention is simplified, compared to methods that involve separate stretching of the film and the strands. The simplified process is important for ensuring quality and productivity for high speed manufacturing operations. Also, the method of the invention is designed to work on a converting line using film and strand material supplied from melt tanks as well as off-line where strand and/or film roll material may be extruder processable.

Tension and retraction properties of the composites can be tuned by varying the ratio of strands to film amount as well as controlling the amount of stretch of the single unit composite. Tension may also be controlled and enhanced through selection of the film composition, selection of the strand composition, selection of the substrate, the add-on rate, and combinations of any of these controlling factors, without risking web breakage problems and without encountering strand misalignment issues.

With the foregoing in mind, it is a feature and advantage of the invention to provide elastic composites and elastic composite laminates having controlled tension. The invention also includes garments having the elastic composite laminates around one or more openings of the garments. The invention further includes methods of making such elastic composites and elastic composite laminates.

### BRIEF DESCRIPTION OF THE DRAWINGS

Fig. 1 is a plan view of an elastic composite of the invention.
Fig. 2 is a plan view of another embodiment of an elastic composite of the invention.
Fig. 3 is a cross-sectional view, taken along line 3-3 of Fig. 1, of another embodiment of an elastic composite of the invention.
Fig. 4 illustrates one embodiment of the method of the invention.
Fig. 5 is a plan view of an elastic composite laminate of the invention.
Fig. 6 is a cross-sectional view, taken along line 6-6 of Fig. 5, of another embodiment of an elastic composite laminate of the invention.
Fig. 7 is a schematic view of another embodiment of the method of the invention.
Fig. 8 is a perspective view of a garment having an elastic composite laminate around the leg openings and waist opening.
Fig. 9 is a perspective view of a medical garment having an elastic composite laminate around the neck opening and the wrist openings.
Fig. 10 is a perspective view of an industrial workwear garment having an elastic composite laminate around the neck opening and the arm openings.
Fig. 11 is a perspective view of an industrial workwear garment having an elastic composite laminate around the waist opening and the ankle openings.
Fig. 12 illustrates another representative process for making the elastic composites and elastic composite laminates of the invention.
Fig. 13 is a schematic view of yet another process for making the elastic composites and elastic composite laminates of the invention.
Fig. 14 is a schematic view of another embodiment of the method of the invention.
Fig. 15 is a schematic view of a lattice-design roller that can be used to produce a mesh layer in yet another embodiment of the invention.
Fig. 16 is a schematic view of one embodiment of a combined NBL and VFL machine that may be used in the method of the invention.
Fig. 17 is a schematic view of another embodiment of a combined NBL and VFL machine that may be used in the method of the invention.

### DEFINITIONS

Within the context of this specification, each term or phrase below will include the following meaning or meanings.

"Bonded" refers to the joining, adhering, connecting, attaching, or the like, of at least two elements. Two elements will be considered to be bonded together when they are bonded directly to one another or indirectly to one another, such as when each is directly bonded to intermediate elements.

"Elastic tension" refers to the amount of force per unit width required to stretch an elastic material (or a selected zone thereof) to a given percent elongation.

"Elastomeric" and "elastic" are used interchangeably to refer to a material or composite that is generally capable of recovering its shape after deformation when the deforming force is removed. Specifically, as used herein, elastic or elastomeric is meant to be that property of any material which, upon application of a biasing force, permits the material to be stretchable to a stretched biased length which is at least about 50 percent greater than its relaxed unbiased length, and that will cause the material to recover at least 40 percent of its elongation upon release of the stretching force. A hypothetical example which would satisfy this definition of an elastomeric material would be a 2.5 cm (1 inch) sample of a material which is elongatable to at least 3.8 cm (1.50 inches) and which, upon being elongated to 3.8 cm (1.50 inches) and released, will recover to a length of less than 3.3 cm (1.30 inches). Many elastic materials may be stretched by much more than 50 percent of their relaxed length, and many of these will recover to substantially their original relaxed length upon release of the stretching force.

"Elongation" refers to the capability of an elastic material to be stretched a certain distance, such that greater elongation refers to an elastic material capable of being stretched a greater distance than an elastic material having lower elongation.

"Extruded" refers to a material that is processed through an extrusion die or a slot coat die connected to an extruder or a melt tank.

"Film" refers to a thermoplastic film made using a film extrusion process, such as a cast film or blown film extrusion process. The term includes apertured films, slit films, and other porous films which constitute liquid transfer films, as well as films which do not transfer liquid.

"Garment" includes personal care garments, medical garments, and the like. The term "disposable garment" includes garments which are typically disposed of after 1-5 uses. The term "personal care garment" includes diapers, training pants, swim wear, absorbent underpants, adult incontinence products, feminine hygiene products, and the like. The term "medical garment" includes medical (i.e. protective and/or surgical) gowns, caps, gloves, drapes, face masks, and the like. The term "industrial workwear garment" includes laboratory coats, cover-alls, and the like.

"Layer" when used in the singular can have the dual meaning of a single element or a plurality of elements.

"Machine direction" as applied to a film, refers to the direction on the film that was parallel to the direction of travel of the film as it left the extrusion or forming apparatus. If the film passed between nip rollers or chill rollers, for instance, the machine direction is the direction on the film that was parallel to the surface movement of the rollers when in contact with the film. "Cross direction" refers to the direction perpendicular to the machine direction. Dimensions measured in the cross direction are referred to as "width" dimensions, while dimensions measured in the machine direction are referred to as "length" dimensions.

"Meltblown fiber" refers to fibers formed by extruding a molten thermoplastic material through a plurality of fine, usually circular, die capillaries as molten threads or filaments into converging high velocity gas (e.g., air) streams which attenuate the filaments of molten thermoplastic material to reduce their diameter, which may be to microfiber diameter. Thereafter, the meltblown fibers are carried by the high velocity gas stream and are deposited on a collecting surface to form a web of randomly dispersed meltblown fibers. Such a process is disclosed for example, in U.S. Patent 3,849,241 to Butin et al. Meltblown fibers are microfibers which may be continuous or discontinuous, are generally smaller than about 0.6 denier, and are generally self bonding when deposited onto a collecting surface.

"Mutually-reinforced" refers to a laminate in which each component provides reinforcement to the other component(s).

"Nonwoven" and "nonwoven web" refer to materials and webs of material having a structure of individual fibers or filaments which are interlaid, but not in an identifiable manner as in a knitted fabric. The terms "fiber" and "filament" are used herein interchangeably. Nonwoven fabrics or webs have been formed from many processes such as, for example, meltblowing processes, spunbonding processes, air laying processes, and bonded carded web processes. The basis weight of nonwoven fabrics is usually expressed in ounces of material per square yard (osy) or grams per square meter (gsm) and the fiber diameters are usually expressed in microns. (Note that to convert from osy to gsm, multiply osy by 33.91.)

"Polymers" include, but are not limited to, homopolymers, copolymers, such as for example, block, graft, random and alternating copolymers, terpolymers, etc. and blends and modifications thereof. Furthermore, unless otherwise specifically limited, the term "polymer" shall include all possible geometrical configurations of the material. These configurations include, but are not limited to isotactic, syndiotactic and atactic symmetries.

"Spunbondfiber" refers to small diameter fibers which are formed by extruding molten thermoplastic material as filaments from a plurality of fine capillaries of a spinnerette having a circular or other configuration, with the diameter of the extruded filaments then being rapidly reduced as taught, for example, in U.S. Patent 4,340, 563 to Appel et al., and U.S. Patent 3,692, 618 to Dorschner et al., U.S. Patent 3,802, 817 to Matsuki et al., U.S. Patents 3, 338, 992 and 3,341, 394 to Kinney, U.S. Patent 3,502, 763 to Hartmann, U.S. Patent 3,502, 538 to Petersen, and U.S. Patent 3,542, 615 to Dobo et al. Spunbond fibers are quenched and generally not tacky when they are deposited onto a collecting surface.

Spunbond fibers are generally continuous and often have average deniers larger than about 0.3, more particularly, between about 0.6 and 10.

"Strand" refers to an article of manufacture whose width is less than a film and is suitable for incorporating into a film, according to the present invention.

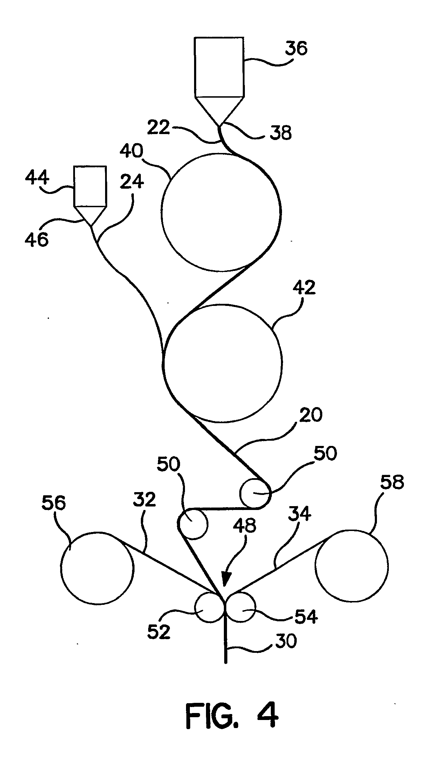

"Stretchable" means that a material can be stretched, without breaking, by at least 50% (to at least 150% of its initial (unstretched) length) in at least one direction, suitably by at least 100% (to at least 200% of its initial length), desirably by at least 150% (to at least 250% of its initial length). The term includes elastic materials as well as materials that stretch but do not significantly retract. The percentage stretch of strands and films is calculated by the percentage difference between a primary chill roll speed and a final nip roll speed. For example, in Fig. 4, if the first chill roller 40 is running at a speed of x and the nip rollers 52 and 54 are running at a speed of 6x, the strands and/or film being stretched between the first chill roller 40 and the nip rollers 52,54 are being stretched 600%.

"Thermoplastic" describes a material that softens and flows when exposed to heat and which substantially returns to a nonsoftened condition when cooled to room temperature.

"Thermoset" describes a material that is capable of becoming permanently cross-linked, and the physical form of the material cannot be changed by heat without the breakdown of chemical bonds.

"Vertical filament stretch-bonded laminate" or "VF SBL" refers to a stretch-bonded laminate made using a continuous vertical filament process, as described herein.

These terms may be defined with additional language in the remaining portions of the specification.

### DETAILED DESCRIPTION OF THE PRESENTLY PREFERRED EMBODIMENTS

The present invention is directed to elastic composites and elastic composite laminates having superior elastic and adhesion properties. The invention is also directed to methods of making such composites and laminates, as well as garments having one or more elasticized openings including an elastic composite laminate attached to a substrate of the garment around, or adjacent, the opening. For example, disposable absorbent articles are known to contain elasticized leg cuffs, elasticized waist portions, and elasticized fastening tabs. The elastic composites and laminates of this invention may be applied to any suitable article to form such elasticized areas.

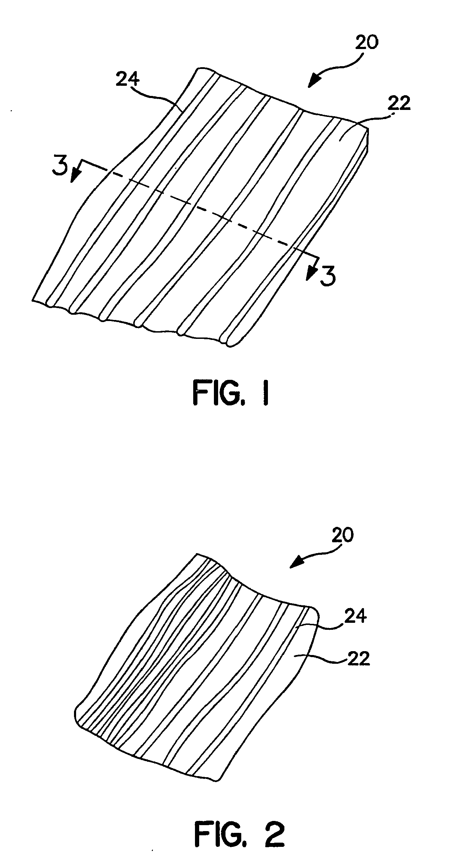

As shown in Fig. 1, an elastomeric composite 20 of the invention includes an elastomeric adhesive film 22 with a number of elastic reinforcing strands 24 adhered to and partially embedded therein.

Tension within the elastomeric composite 20 may be controlled through percentage stretch of the strands 24 prior to adhesion to the elastomeric adhesive film 22, through percentage stretch of the film 22 prior to adhesion to the strands 24, and/or through the amount of strand add-on or thickness, with greater stretch and greater add-on or thickness each resulting in higher tension. Tension can also be controlled through selection of the film composition, selection of the strand composition, and/or by varying strand geometries and/or spacing between strands. It will be appreciated that the strands 24 may be laid out periodically, non-periodically, and in various spacings, groupings, and sizes, according to the effect desired from the composite 20 and the use to which it is put.

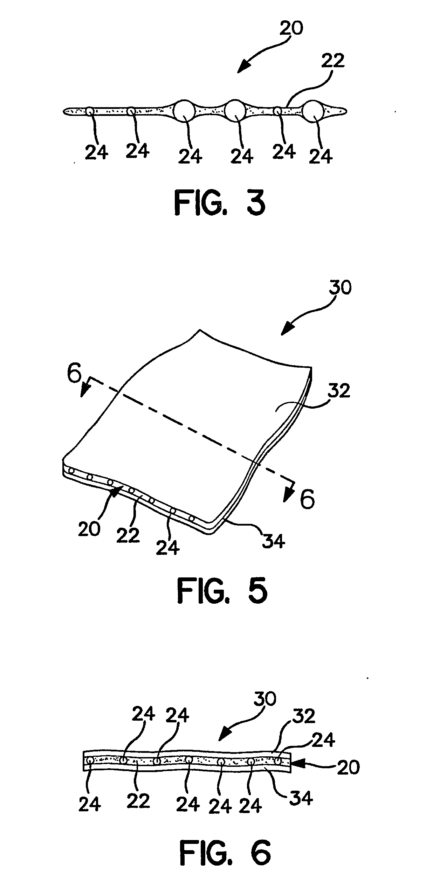

As shown in Fig. 2, for example, a group of strands 24 in one region of the composite 20 can be spaced apart much more closely than another group of strands 24, resulting in greater tension in the region in which the strands 24 are more closely spaced. For instance, the more closely spaced strands may be positioned closer to an edge of a garment opening while the more distantly spaced strands may be positioned inward from the edge to provide a sort of transitional elasticized area. As another example, Fig. 3 illustrates a cross-sectional view of the composite 20 having unequally sized elastic strands 24 with some strands having a larger diameter, and thus higher tension, than others. While referred to as being of different diameter, it will be appreciated that the strands 24 need not be circular in cross-section within the context of this invention. Furthermore, the strands 24 of different size or composition may be intermingled within groupings in regular or irregular patterns.

The elastomeric adhesive film 22 is suitably made up of an elastomeric, hot melt, pressure-sensitive adhesive having an adhesive bond strength, as determined by the test method set forth below, of at least 50 grams force per inch (2.54 cm) width, suitably of at least 100 grams force per inch (2.54 cm) width, alternatively of at least 300 grams force per inch (2.54 cm) width, alternatively of at least from about 100 grams force per inch (2.54 cm) width to about 400 grams force per inch width. An example of a suitable elastomeric adhesive film 22 may be made up of 35 wt% PICOLYTE S115 and 65 wt% KRATON G2760. The elastomeric, hot melt, pressure-sensitive adhesive may be applied to a chill roll or similar device, in the form of a strand or ribbon. The strand or ribbon is then minimally stretched and thinned to form the film 22. The film suitably has a thickness of about 0.001 inch (0.025 mm) to about 0.05 inch (1.27 mm), alternatively of from about 0.001 inch (0.025 mm) to about 0.01 inch (0.25 mm), and a width of from about 0.05 inch (1.27 mm) to about 3.0 inches (7.62 cm), alternatively of from about 0.5 inch (1.27 cm) to about 1.5 inches (3.81 cm). The elastomeric, adhesive film 22 may also be capable of imparting barrier properties in an application.

Suitable elastomeric, hot melt, pressure-sensitive adhesives from which the elastomeric adhesive film 22 may be made include elastomeric polymers, tackifying resins, plasticizers, oils and antioxidants.

One particular formulation of the elastomer adhesive film 22 includes a base polymer and a tackifier resin. The composition may also include additional additives. The choice of polymer and tackifier is important, as is the ratio of polymer or copolymers to tackifier. Another important consideration is the ratio of additives to tackifier.

The base polymer suitably has a styrene content of between about 15% and about 45%, or between about 18% and about 30%, by weight of the base polymer. The base polymer may achieve the styrene content either by blending different polymers having different styrene co-monomer levels or by including a single base polymer that has the desired styrene co-monomer level. Generally, the higher the styrene co-monomer level is, the higher the tension is.

The base polymer may include polystyrene-polyethylene-polypropylene-polystyrene (SEPS) block copolymer, styrene-isoprene-styrene (SIS), styrene-butadiene-styrene (SBS) block copolymer, as well as combinations of any of these. One example of a suitable SEPS copolymer is available from Kraton Polymers of Belpre, Ohio, under the trade designation KRATON® G 2760. One example of a suitable SIS copolymer is available from Dexco, a division of Exxon-Mobil, under the trade designation VECTOR™. Suitably, the film composition includes the base polymer in an amount between about 30% and about 65% by weight of the composition.

The tackifier may include hydrocarbons from petroleum distillates, rosin, rosin esters, polyterpenes derived from wood, polyterpenes derived from synthetic chemicals, as well as combinations of any of these. A key element of the film composition is a tackifier. An example of a suitable tackifier is available from Hercules Inc. of Wilmington, Delaware, under the trade designation PICOLYTE™ S 115. Suitably, the composition includes the tackifier in an amount between about 30% and about 70% by weight of the composition.

Other additives may be included in the film composition as well. In addition to the adhesion provided by the tackifier, various additives may provide instantaneous surface tackiness and pressure sensitive characteristics as well as reduced melt viscosity. One example of a particularly suitable low softening point additive is PICOLYTE™ S25 tackifier, available from Hercules Inc., having a softening point in a range around 25 degrees Celsius, or paraffin wax having a melting point of about 65 degrees Celsius may also be used.

Additionally, an antioxidant may be included in the film composition, suitably in an amount between about 0.1% and about 1.0% by weight of the composition. One example of a suitable antioxidant is available from Ciba Specialty Chemicals under the trade designation IRGANOX™ 1010.

The elastomeric adhesive film 22 suitably has an elongation of at least 50 percent, alternatively of at least 150 percent, alternatively of from about 50 percent to about 200 percent, and a tension force of less than about 400 grams force per inch (2.54 cm) width, alternatively of less than about 275 grams force per inch (2.54 cm) width, alternatively of from about 100 grams force per inch (2.54 cm) width to about 250 grams force per inch (2.54 cm) width. Tension force, as used herein, is determined one minute after stretching the film to 100% elongation. A method for determining elongation is described in detail below.

The elastomeric adhesive film 22 is capable not only of introducing a degree of elasticity to facing materials but is also capable of providing a construction adhesive function. That is, the film 22 adheres together the facing materials or other components with which it is in contact. It is also possible that the film does not constrict upon cooling but, instead, tends to retract to approximately its original dimension after being elongated during use in a product.

Materials suitable for use in preparing the elastic reinforcing strands 24 include raw polymers, a mixture of polymers, as well as tackified polymers. More specifically, the elastic reinforcing strands 24 may include diblock, triblock, tetrablock, or other multi-block elastomeric copolymers such as olefinic copolymers, including ethylenepropylene-diene monomer (EPDM), styrene-isoprene-styrene (SIS), styrene-butadiene-styrene (SBS), styrene-ethylene/butylene-styrene (SEBS), or styrene-ethylene/propylene-styrene (SEPS), which may be obtained from the Kraton Polymers of Belpre, Ohio, under the trade designation KRATON® elastomeric resin or from Dexco, a division of Exxon-Mobil, under the trade designation VECTOR® (SIS polymers); polyurethanes, including those available from E. I. Du Pont de Nemours Co., under the trade name LYCRA® polyurethane; polyamides, including polyether block amides available from Ato Chemical Company, under the trade name PEBAX® polyether block amide; polyesters, such as those available from E. I. Du Pont de Nemours Co., under the trade name HYTREL® polyester; polyisoprene; cross-linked polybutadiene; and single-site or metallocene-catalyzed polyolefins having density less than about 0.89 grams/cubic centimeter, available from Dow Chemical Co. under the trade name AFFINITY®. The elastic reinforcing strands 24 may also include a tackifier. The tackifier may include hydrocarbons from petroleum distillates, rosin, rosin esters, polyterpenes derived from wood, polyterpenes derived from synthetic chemicals, as well as combinations of any of these.

A number of block copolymers can also be used to prepare the elastic reinforcing strands 24 used in this invention. Such block copolymers generally include an elastomeric midblock portion B and a thermoplastic endblock portion A. The block copolymers may also be thermoplastic in the sense that they can be melted, formed, and resolidified several times with little or no change in physical properties (assuming a minimum of oxidative degradation). Alternatively, the elastic strands 24 can be made of a polymer that is not thermally processable, such as LYCRA® spandex, available from E. I. Du Pont de Nemours Co., or cross-linked natural rubber in film or fiber form. Thermoset polymers and polymers such as spandex, unlike the thermoplastic polymers, once cross-linked cannot be thermally processed, but can be obtained on a spool or other form and can be stretched and applied to the strands in the same manner as thermoplastic polymers. As another alternative, the elastic strands 24 can be made of a thermoset polymer, such as AFFINITY®, available from Dow Chemical Co., that can be processed like a thermoplastic, i.e. stretched and applied, and then treated with radiation, such as electron beam radiation, gamma radiation, or UV radiation to cross-link the polymer, or use polymers that have functionality built into them such that they can be moisture-cured to cross-link the polymer, thus resulting in a polymer and the enhanced mechanical properties of a thermoset.

Endblock portion A may include a poly(vinylarene), such as polystyrene. Midblock portion B may include a substantially amorphous polyolefin such as polyisoprene, ethylene/propylene polymers, ethylene/butylenes polymers, polybutadiene, and the like, or mixtures thereof.

Suitable block copolymers useful in this invention include at least two substantially polystyrene endblock portions and at least one substantially ethylene/butylenes mid-block portion. A commercially available example of such a linear block copolymer is available from Kraton Polymers under the trade designation KRATON® G1657 elastomeric resin. Another suitable elastomer is KRATON® G2760. Yet another suitable elastomer is an SIS triblock copolymer available from Dexco, a division of Exxon-Mobil, under the trade designation VECTOR®.

The elastic reinforcing strands 24 may also contain blends of elastic and inelastic polymers, or of two or more elastic polymers, provided that the blend exhibits elastic properties. The strands 24 are substantially continuous in length. The strands 24 may have a circular cross-section but, as previously mentioned, may alternatively have other cross-sectional geometries such as elliptical, rectangular, triangular or multi-lobal. In one embodiment, one or more of the elastic reinforcing strands 24 may be in the form of elongated, rectangular strips produced from a film extrusion die having a plurality of slotted openings.

The ratio of film to strands in the composite may be adjusted to control tension in the composite the elastic strands account for between about 5% and about 50%, or between about 10% and about 35%, or between about 15% and about 25% by weight of the composite, namely the combination of the film and the elastic strands.

Fig. 4 illustrates a method and apparatus for making a strand-reinforced elastic composite as described above. While Fig. 4 illustrates a composite VF SBL process it will be appreciated that other processes consistent with the present invention may be used. A melt tank 36 using a slotted film die 38, for example, produces the elastomeric adhesive film 22 which is cast onto a first chill roll 40 and passed to a second roll 42, such as a second chill roll. An extruder 44 produces reinforcing strands of elastic material 24 through a filament die 46. The strands 24 are extruded onto the film 22 while the film is in contact with the second roll 42, thereby creating a strand-reinforced composite 20 on the second roll 42. The composite 20 is stretched as a single unit while conveyed vertically towards a nip 48 by one of more fly rollers 50. The nip 48 is formed by opposing first and second nip rollers 52,54. For example, the composite may be stretched between about 200% and about 1200%, or between about 400% and about 1000%. Another process parameter is the add-on rate. More specifically, the elastic strands may be adhered to, and partially embedded in, the elastomeric adhesive film at an add-on rate of between about 5 and about 50 grams per square meter before stretching.

By stretching the composite 20 as a unit, namely with the film 22 and strands 24 combined, as opposed to stretching the film and the strands separately, a more robust manufacturing method can be carried out. More particularly, the strands 24 and the film 22 reinforce one another, thereby minimizing or avoiding film breakage and strand breakage. Thus, the composite 20 may be considered a mutually-reinforced strand/film composite. Strand alignment problems are also minimized or avoided because the method of the invention forces the strands 24 to align themselves on the film 22 in the machine direction without moving in the cross direction or breaking. The alignment of the strands 24 also reinforces the film 22, especially along the edges of the film.

The mutually reinforced strand and film combination allows the composite 20 to be stretched considerably during the process, and the web speed can be run at a considerably high speed. Furthermore, the process is essentially simplified, which is important for ensuring quality and productivity for high-speed manufacturing operations. The ability to control processability is especially critical for converting line operations where start/stop situations need to be minimized for economic reasons.

A strand-reinforced elastomeric composite laminate 30, or mutually reinforced elastomeric strand/film composite laminate, may be formed by laminating a first facing sheet 32 and a second facing sheet 34 to opposite surfaces of the stretched elastomeric composite 20. Examples of such laminates 30 are illustrated in Figs. 5 and 6. Facing materials may be formed using conventional processes, including the spunbond and meltblowing processes described in the DEFINITIONS. For example, the facing sheets 32, 34 may each include a nonwoven web, such as a spunbonded web having a basis weight of about 3.4 to 135.6 grams per square metre (gsm) (0.1-4.0 ounces per square yard (osy)), suitably 6.8 to 67.8 gsm (0.2-2.0 osy), or about 13.6 to 20.3 gsm (0.4-0.6 osy). As another example, the facing sheets 32,34 may each include a non-porous polyolefin film, such as outer cover material, or a combination of film and spunbond material. One or more facing sheets 32,34 may be present in the laminate 30. The facing sheets 32,34 may include the same or similar materials or different materials. Examples of suitable types of facing sheet 32,34 combinations include at least one sheet of spunbond and at least one sheet of film, or two sheets of film, or two sheets of spunbond.

If the facing sheets 32,34 are to be applied to the composite 20 without first being stretched, the facing sheets may or may not be capable of being stretched in at least one direction in order to produce an elasticized area. For example, the facing sheets 32,34 could be necked, or gathered, in order to allow them to be stretched after application of the elastic composite 20. Various post treatments, such as treatment with grooved rolls, which alter the mechanical properties of the material, are also suitable for use.

In order to form the elastic composite laminate 30, first and second rolls 56 and 58, respectively, of spunbond facing material or other suitable facing material are fed into the nip 48 on either side of the elastic composite, as shown in Fig. 4, and are bonded by the adhesive present in the elastic composite. The facing material might also be made in situ rather than unrolled from previously-made rolls of material. While illustrated as having two lightweight gatherable spunbond facings, it will be appreciated that only one facing material, or various types of facing materials, may be used.

Tension and retraction properties within the elastomeric composite 20 or laminate 30 may be controlled, or tuned, during the process through percentage stretch of the composite prior to adhesion to the facing sheets, through the ratio of strands to film, and/or through the amount of strand add-on or thickness, with greater stretch and greater add-on or thickness each resulting in higher tension. Tension can also be controlled through selection of the film composition, selection of the strand composition, substrate selection, and/or by varying strand geometries and/or spacing between strands.

The method of the invention is designed to work on the converting line using film and/or strand material supplied from melt tanks as well as off-line where strands and/or film material obtained on a supply roll, or other method of storage, may be extruder processable.

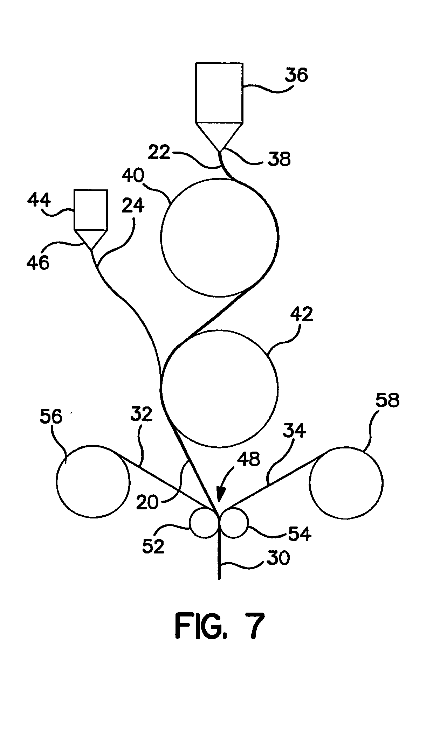

Fig. 7 illustrates a VF SBL process in which no fly rollers 50 are used. Instead, the elastomeric adhesive film 22 is extruded onto chill roller 40 and is passed to chill roller 42. The strands 24 are extruded onto chill roller 42 on top of the film 22 thereby forming the strand-reinforced composite 20. The composite 20 is stretched between the chill rollers 42 and the nip 48. Except for the lack of fly rollers, the processes of Figs. 4 and 7 are similar. In either case, the strands 24 and the elastomeric adhesive film 22 together are laminated between a first facing layer 32 and a second facing layer 34 at the nip 48.

The resulting elastic composites and elastic composite laminates are considerably strong and resistant to breaking and are therefore particularly useful in providing elasticity in personal care absorbent garments, described in greater detail below. The elastic composite laminates provide better fit and comfort, as well as less leakage and more tension control compared to conventional elastic strands and elastic strand laminates. The laminates of this invention are less likely to undergo tension decay or delamination compared to similar laminates lacking the reinforcing strands, as demonstrated in the example below. The tension retaining capability of the laminates insures product performance and reduces or eliminates leakage during use. Also, by incorporating the strands within the film, the tension load on the resulting laminate is distributed among the entire width of the elastic composite instead of being concentrated on the individual strands. Furthermore, the reinforcing strands enable the composite tension to be tunable while preserving the soft feel and aesthetic properties of the laminate. Thus, elastic composite laminates can be produced with a desired fit or gasket-like quality without causing red marks on a wearer's skin due to excessive tension, while preserving the soft and gentle feel and improved adhesion of the laminate. Additionally, because the strands are incorporated within the film, the elastic composite laminate can be die-cut into virtually any shape to provide enhanced fit and comfort without losing tension.

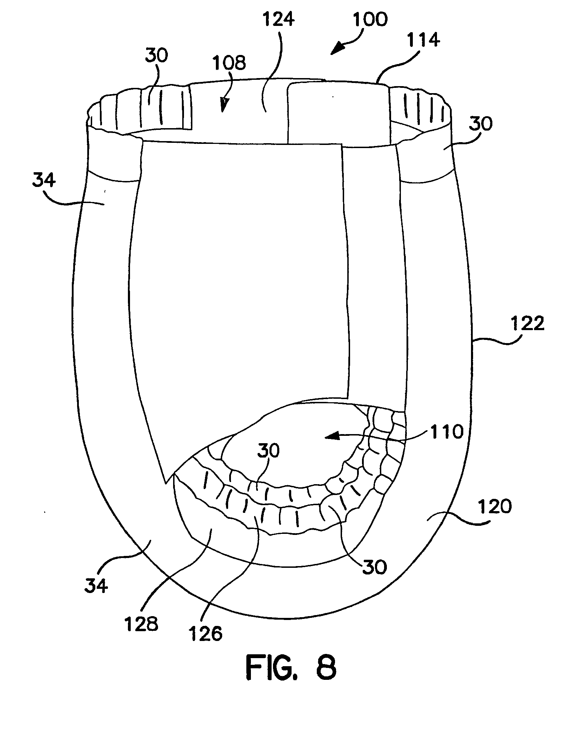

A garment 100 including such composites or laminates suitably has one or more elasticized openings. The opening or openings may include a neck opening 102, a wrist opening 104, an arm opening 106, a waist opening 108, a leg opening 110, and/or an ankle opening 112, as shown in Figs. 8-11. The opening or openings include an elastic composite laminate 30 attached to a substrate 34 of the garment 100 around, or adjacent, the opening. More particularly, the laminate may include two facing sheets 32, one on each surface of the elastic composite 20, and the laminate may then be attached to the garment with one of the facing sheets 32 bonded to the substrate 34. Alternatively, the laminate may include one facing sheet on one surface of the elastic composite 20, and the substrate 34 of the garment may serve as a second facing sheet on a second surface of the elastic composite 20.

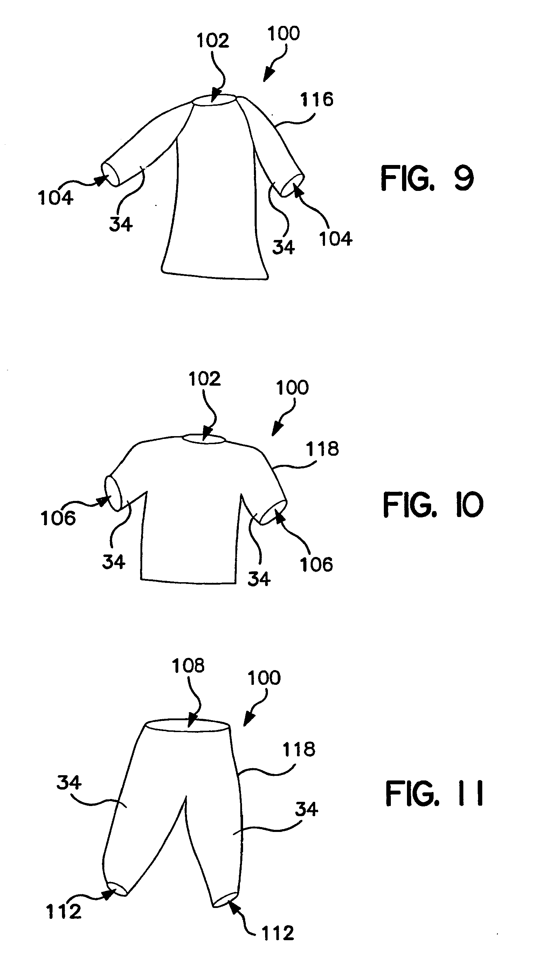

The garment 100 may be a personal care garment 114 (Fig. 8), medical garment 116 (Fig. 9), industrial workwear garment 118 (Figs. 10 and 11), or the like. More particularly, the garment 100 may be a diaper, training pants, swim wear, absorbent underpants, adult incontinence product, feminine hygiene product, protective medical gown, surgical medical gown, cap, gloves, drape, face mask, laboratory coat, or coveralls, for example. For ease of explanation, the following description is in terms of a pant-like garment 100, such as a child's training pant, having the elastic composite laminate 30 adjacent each of the leg openings 110.

Referring to Fig. 8, a disposable absorbent garment 100, such as a child's training pant, includes a chassis 120. The chassis 120 suitably includes an outer cover 122, a body side liner 124, and an absorbent assembly (not shown) positioned between the outer cover and the body side liner. The chassis 120 defines a waist opening 108 and two leg openings 110. An elastomeric composite laminate 30 is applied to the chassis 120 adjacent each of the leg openings 110, and possibly adjacent the waist opening 108 as well, to provide elasticity. Tension in the elastomeric composite laminate 30 may be controlled, as described above.

The substrate 34 is suitably the outer cover 122, the body side liner 124, or any other material of the garment 100 that forms the opening to which the elastomeric composite laminate 30 is attached. The outer cover 122, for example, desirably includes a material that is substantially liquid impermeable, and can be elastic, stretchable or nonstretchable. The outer cover 122 can be a single layer of liquid impermeable material, but desirably includes a multi-layered laminate structure in which at least one of the layers is liquid impermeable. For instance, the outer cover 122 can include a liquid permeable outer layer and a liquid impermeable inner layer that are suitably joined together by a laminate adhesive (not shown). Suitable laminate adhesives, which can be applied continuously or intermittently as beads, a spray, parallel swirls, or the like, can be obtained from Findley Adhesives, Inc., of Wauwatosa, Wisconsin, U.S.A., or from National Starch and Chemical Company, Bridgewater, New Jersey, U.S.A. The liquid permeable outer layer can be any suitable material and desirably one that provides a generally cloth-like texture. One example of such a material is a 20 gsm (grams per square meter) spunbond polypropylene nonwoven web. The outer layer may also be made of those materials of which the body-side liner 124 is made. While it is not a necessity for the outer layer to be liquid permeable, it is desired that it provides a relatively cloth-like texture to the wearer.

The inner layer of the outer cover 122 can be both liquid and vapor impermeable, or can be liquid impermeable and vapor permeable. The inner layer is desirably manufactured from a thin plastic film, although other flexible liquid impermeable materials may also be used. The inner layer, or the liquid impermeable outer cover 122 when a single layer, prevents waste material from wetting articles, such as bedsheets and clothing, as well as the wearer and care giver. A suitable liquid impermeable film for use as a liquid impermeable inner layer, or a single layer liquid impermeable outer cover 122, is a 0.2 millimeter polyethylene film commercially available from Huntsman Packaging of Newport News, Virginia, U.S.A. If the outer cover 122 is a single layer of material, it can be embossed and/or matte finished to provide a more cloth-like appearance. The liquid impermeable material can permit vapors to escape from the interior of the garment, while still preventing liquids from passing through the outer cover 122. A suitable "breathable" material is composed of a microporous polymer film or a nonwoven fabric that has been coated or otherwise treated to impart a desired level of liquid impermeability. A suitable microporous film is a PMP-1 film material commercially available from Mitsui Toatsu Chemicals, Inc., Tokyo, Japan, or an XKO-8044 polyolefm film commercially available from 3M Company, Minneapolis, Minnesota.

The body side liner 124 suitably overlies the outer cover 122 and absorbent assembly, and may but need not have the same dimensions as the outer cover 122. The body side liner 124 is desirably compliant, soft feeling, and non-irritating to the wearer's skin. Further, the body side liner 124 can be less hydrophilic than the absorbent assembly, to present a relatively dry surface to the wearer and permit liquid to readily penetrate through its thickness.

The body side liner 124 can be manufactured from a wide selection of web materials, such as synthetic fibers (for example, polyester or polypropylene fibers), natural fibers (for example, wood or cotton fibers), a combination of natural and synthetic fibers, porous foams, reticulated foams, apertured plastic films, or the like. Various woven and nonwoven fabrics can be used for the body side liner 124. For example, the body side liner can be composed of a meltblown or spunbonded web of polyolefin fibers. The body side liner can also be a bonded-carded web composed of natural and/or synthetic fibers. The body side liner can be composed of a substantially hydrophobic material, and the hydrophobic material can, optionally, be treated with a surfactant or otherwise processed to impart a desired level of wettability and hydrophilicity. For example, the material can be surface treated with about 0.45 weight percent of a surfactant mixture including AHCOVEL^{®} N-62 available from Uniqema Inc., a division of ICI of New Castle, Delaware, U.S.A. and GLUCOPON^{®} 220UP available from Cognis Corporation of Ambler, Pennsylvania, and produced in Cincinnati, Ohio, in an active ratio of 3:1.

A suitable liquid permeable body side liner 124 is a nonwoven bicomponent web having a basis weight of about 27 gsm. The nonwoven bicomponent can be a spunbond bicomponent web, or a bonded carded bicomponent web. Suitable bicomponent staple fibers include a polyethylene/polypropylene bicomponent fiber available from CHISSO Corporation, Osaka, Japan. In this particular bicomponent fiber, the polypropylene forms the core and the polyethylene forms the sheath of the fiber. Other fiber orientations are possible, such as multi-lobe, side-by-side, end-to-end, or the like.

Pant-like garments, such as the training pant shown in Fig. 8, may include a pair of containment flaps 126 which are configured to provide a barrier to the transverse flow of body exudates. The containment flaps 126 can serve as the substrate 34 in the garment of the invention such that the elastomeric composite 20 attaches a facing sheet 32 to each containment flap 126. The elastomeric composite laminate 30 along the edge of each containment flap 126 defines an unattached edge which assumes an upright, generally perpendicular configuration in at least the crotch region of the training pant to form a seal against the wearer's body. Suitable constructions and arrangements for the containment flaps are generally well known to those skilled in the art and are described in U.S. Patent 4,704, 116 issued November 3,1987, to Enloe. Additionally, the elastomeric composite 20 may attach a facing_sheet 32 around each leg opening 110 to form leg cuffs 128. In certain embodiments, the elastomeric composite laminate 30 may be present in the containment flaps, the leg cuffs, or both the containment flaps and the leg cuffs.

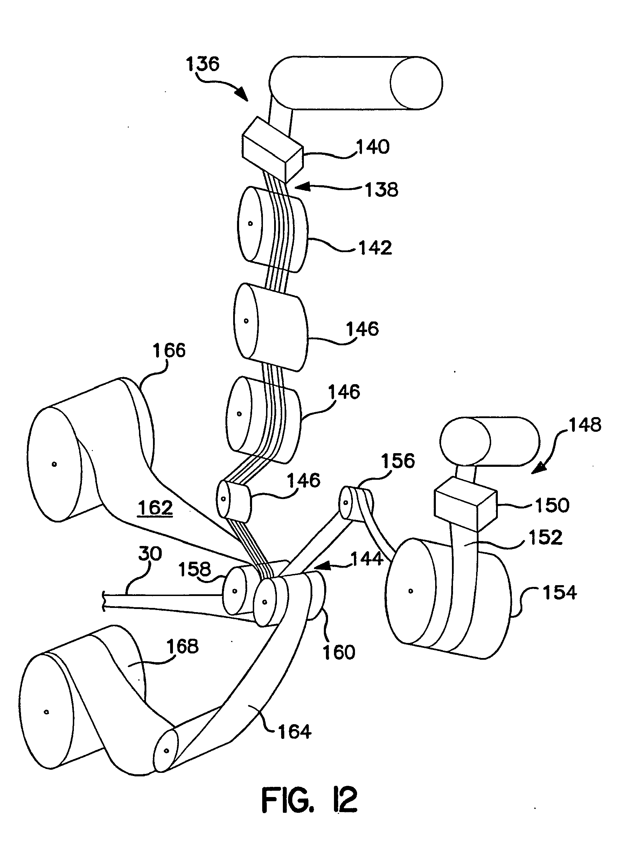

Fig. 12 illustrates another composite VF SBL method and apparatus for making an elastic composite laminate 30 of the invention. A first extruder 136 produces reinforcing strands of elastic material 138 through a filament die 140. The strands 138 are fed to a first chill roller 142 and stretched while conveyed vertically towards a nip 144 by one or more first fly rollers 146 in the strand-producing line. As in the earlier described method, the elastic strands may be adhered to, and partially embedded in, the elastomeric adhesive film at an add-on rate of between about 5 and about 50 grams per minute before stretching.

A second extruder 148 using a slotted film die 150 produces the elastomeric adhesive film 152, which is fed onto a second chill roller 154 and conveyed to one or more second fly rollers 156 towards the nip 144. The film 152 may be stretched down to a narrower width and thinned by the second fly rollers 156 during its passage to the nip 144. The nip 144 is formed by opposing first and second nip rollers 158,160. The elastic composite 20 is formed by adhering the strands 138 to the elastomeric adhesive film 152 in the nip 144.

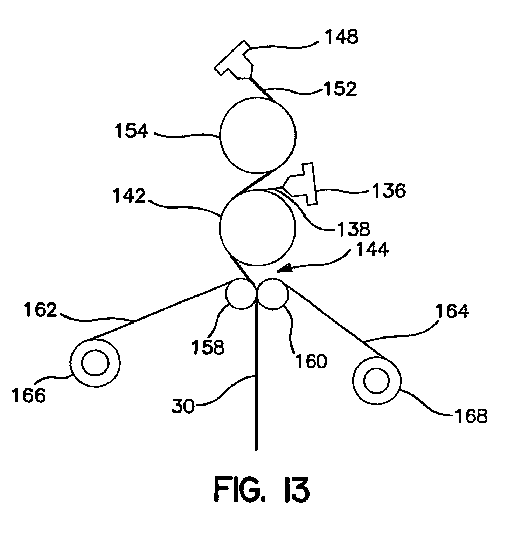

Fig. 13 illustrates another VF SBL process in which no fly rollers 146,156 are used. Instead, the elastomeric adhesive film 152 is extruded onto chill roller 154. The strands 138 are extruded onto chill roller 142 where the strands 138 and the elastomeric adhesive film 152 converge. The strands 138 and the elastomeric adhesive film 152 are stretched between the chill rollers 142, 154 and the nip 144. Except for the lack of fly rollers, the processes of Figs. 12 and 13 are similar. In either case, the strands 138 and the elastomeric adhesive film 152 together are laminated between a first facing layer 162 and a second facing layer 164 at the nip 144.

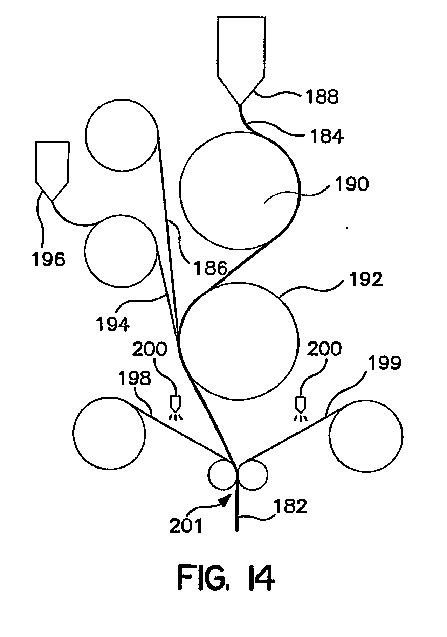

In another embodiment of the invention, illustrated in Fig. 14, a biaxial stretch laminate 182 may be formed by casting a film 184 through a first film die 188 onto a first roll 190, stretching the film, and spinning and stretching a plurality of strands 186 onto the film while the film is stretched. The method may also include thinning, necking, and/or heat-treating the film. The film may also be second-stretched, or stretched a second time. The strands may also be heat-treated and/or second-stretched. Alternatively, ribbons of film rather than strands may be stretched and adhered to the stretched film. As another alternative, both ribbons of film and strands may be adhered to the film. The stretching steps create thinner films or strands, resulting in laminates that are thinner overall. Furthermore, by warming the film 184 on the first roll 190, stretching the film, cooling the film on a second roll 192, and stretching the film again, a necked elastic film may be created.

A second film 194 may be cast through a second film die 196 over the strands or ribbons 186 on top of the first film 184 as the first film is stretched, thereby encapsulating the strands 186 between film layers 184, 194. The second film 194 may instead be a foam, or a foamed film, that is foamed inline on top of the first film and strands. Polyurethane film does not set up right away and would therefore foam easily.

Additionally, one or more facing materials 198, 199 may be laminated to the film or films. The facing material(s) may include any of the previously discussed facing materials, such as a necked material. The laminate 182 resulting from this embodiment of the method of the invention may have different stretch in the machine direction than in the cross direction. Furthermore, the laminates may be "zoned," with different elastic properties along the length and/or width of the laminate which may be created through zoning the film and/or the strands. Additionally, adhesive 200 used to bond the facing materials 198, 199 to the film may be zoned to create additional functionality.

The film 184, 194 in this embodiment need not be limited to elastomeric adhesive films, but instead may include any suitable elastomeric film, or even a foamed elastomer such as polyurethane. The film may be filled with calcium carbonate, for example, or any other suitable filler to impart breathability to the film upon stretching. The strands 186 may include strands of elastomer, for example, such as any of the strand materials described in the previous embodiments.

Most laminates currently have one layer of elastomeric material or at most two that are integrated upon extrusion and not handled separately green before they are integrated together. As used herein, the term "green" refers to a material that has never been rolled up or otherwise placed in storage. The ability to handle the film and strands or film ribbons and strands or strand and foams separately, but in the same process, provides benefits to forming new elastic structures beyond those currently practiced.

Similar to the embodiments described above, by fixing the strands 186 onto the green film 184 the occurrence of strand slippage is greatly reduced. Since the film is pressure-sensitive and tacky when the strands are applied to the film, the need for adhesive between the film 184 and the strands 186 is eliminated, and, in addition, strand slippage is prevented. Additionally, the strands 186 may be at least partially embedded in, or encapsulated by, the layer of green film 184.

Another advantage of this embodiment is that the cast and annealing rolls 190, 192 in the process can be run independent of each other in terms of temperature and speed, which means that the elastic film 184 can be annealed or cooled as desired to introduce properties such as latency, deadening of the elastic, or even enhancing the setting of the elastomer for more immediate elastic properties.

Another benefit of this embodiment is the point at which the facings 198, 199 are introduced into the process. If the first facing 198 is laminated to the elastic film/strand layer on the second roll 192 and the second facing 199 is laminated later at the nip point 201, then a two stretch-to-stop laminate can be generated. A laminate having two stretch-to-stop points has unique properties.

The material resulting from this embodiment of the invention can be used for side panels, ears, waist bands, leg elastics, and the like, or for outer covers incorporating elastic and breathable properties. Any of the elements of this embodiment can be combined with any of the elements of the preceding embodiments.

In yet another embodiment of the invention, the invention includes a multilayer elastic or at least extensible laminate made from film (filled or monolithic) and fibers with possible subsequent lamination to at least one facing material which may be a necked material, a mesh material, or other substrate to form a biaxial stretch laminate. The laminate may have different stretch properties in the machine direction than in the cross direction. The film can be either continuous or discontinuous, such as in the form of ribbons.

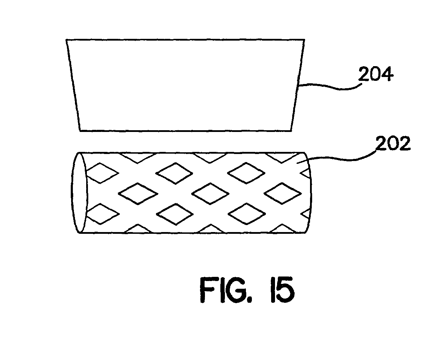

In one particular embodiment, for example, the laminate may include an extensible film, filled with calcium carbonate or other suitable filler to provide breathability upon stretching. The laminate may also include an inherently extensible or elastic nonwoven layer, such as a bicomponent spunbond web, or adhesively laminated layers of such materials. Between the film and the nonwoven layers is an extruded elastic mesh structure, which can be made with an elastomer and can also provide bias stretch due to the structure. An illustration of apparatus for creating the mesh layer is provided in Fig. 15. More particularly, the apparatus includes a patterned roll 202 onto which the elastomer is extruded from a film die 204. This laminate structure has the advantage of using a film layer that can be formulated to meet breathability targets without the additional burden of being elastic by essentially decoupling elastic and breathability requirements for the film. The mesh structure is essentially an extruded layer so there is flexibility in resin choice, mesh structure, and the like.

In another particular embodiment, for example, the laminate may include a breathable cross-direction-extensible film, or a very low basis weight monolithic film, with an elastomer printed onto a surface of the film in stripes or bars. The elastomer provides sufficient stretch and recovery. The film is then laminated to an inherently extensible and/or elastic film.

In yet another particular embodiment, for example, the laminate may include a 3-layer film with outer film layers that are breathable and extensible or elastic and a middle layer of elastic strands between the two outer film layers. Facing sheets are either adhesively or thermally bonded to the outer film layers. Each of the film layers is extruded through a die, with the middle layer extruded through a die designed to create strips or ribbons of elastomer. If the two outer film layers do not fully marry, or bond to one another across the entire contact surface, gaps are formed which may serve as a built-in spacer layer. Alternatively, the outer film layers may be treated with a humectant or water-vapor-absorbing coating or particulate on the surface in contact with the middle layer to minimize water vapor transfer through the laminate, thereby potentially eliminating dampness concerns at high breathability levels.

These laminates are particularly suitable for providing outer covers with breathability, cross-direction extensibility, and possibly dampness control. Any of the elements of this embodiment can be combined with any of the elements of the preceding embodiments.

In still another embodiment of the invention, a machine capable of carrying out both vertical filament laminate (VFL) processes and neck bonded laminate (NBL) processes can be used to carry out any of the methods of the invention. Because the two processes are so similar, this combination machine results in lower capital cost and increased asset flexibility compared to having two separate machines. This machine has unique characteristics that allow it to process machine direction, cross direction and biaxial stretch materials.

Both NBL and VFL laminates may include similar facings, such as spunbond or other suitable nonwoven webs, with the facings in the NBL being neck-stretched. Furthermore, the facings in both the NBL and VFL processes may be adhesively or thermally bonded to an elastic core. The elastic core may be either a cast film for NBL or filament strands for VFL.

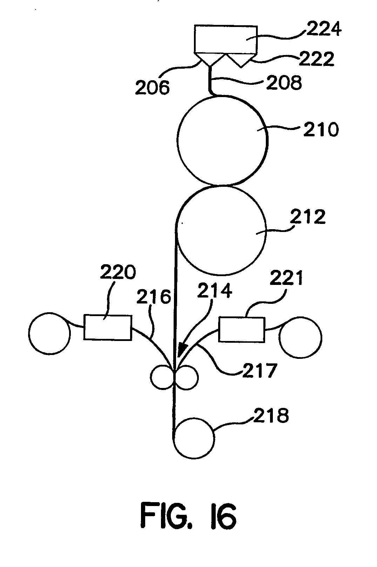

The combination machine is based on a conventional VFL platform including a filament die 206 for extruding the elastic core 208 onto a first chill roll or forming roll 210, and a second roll 212 which passes the elastic core 208 to a nip 214 with the facings 216, 217 applied to the elastic core 208 prior to passing through the nip 214 and onto a winder 218. Additional equipment is added to the VFL platform to enable the production of NBL. The additional equipment includes ovens 220, 221 for neckstretching the facings 216, 217 and a film die 222 mounted adjacent the filament die 206. The similar throughputs of NBL and VFL, based on elastic basis weight and web width, enable the two processes to be combined into one machine while utilizing the same extruder 224 and pellet handling systems, or alternatively, conventional hot-melt equipment such as a melt tank or an extruder. One embodiment of the machine is illustrated in Fig. 16.

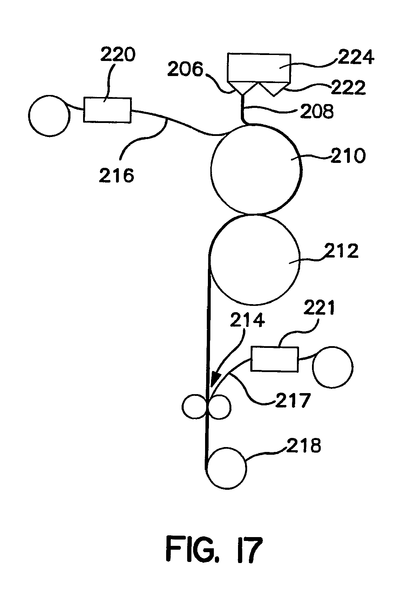

Alternatively, the machine may be set up such that one of the facings 216 is guided onto the first forming roll 210 such that the elastic core 208 is extruded onto the facing 216. This embodiment of the machine is illustrated in Fig. 17.

Any of the elements of this machine can be combined with any of the elements of the preceding embodiments.

### Test Methods

### Adhesive Bond Strength

The adhesive bond strength of the elastomeric adhesive film of the present invention is determined as follows. A test sample of the elastic composite laminate having dimensions of about 2.0 inches (5.08 cm) wide by about 4.0 inches (10.16 cm) long, or as large as possible up to this size, is used for testing. The adhesive bond strength is determined through the use of a tensile tester, such as a SINTECH tensile tester commercially available from the Sintech Co. , Carry, North Carolina, Model No. II. A 90 degree peel adhesion test is run in order to determine the grams of force needed to pull apart the first and second layers of facing sheet of the laminate. Specifically, 1.25 inches (3.175 cm) or more of the 10.16 cm (4-inch) length of the test sample has the first and second layers of facing sheet peeled apart. The first facing sheet is then clamped in the upper jaw of the tensile tester, and the second facing sheet is clamped in the lower jaw of the tensile tester. The tensile tester is set to the following conditions:
Crosshead speed: 300 millimeters per minute
Full-scale lad: 5,000 grams
Start measurements: 10 millimeters
Gauge length (jaw spacings): 1.0 inch (2.54 cm)

The Instron tensile tester is then engaged. The test is terminated after approximately 100 millimeters on a 5.08 by 5.08 cm (2-inch by 2-inch) sample. Twenty data points per second are collected for a total of about 400 data points. The average of these data points is reported as the adhesive bond strength. The results from the tensile tester are normalized to a sample having a width of 2.54 cm (1 inch). At least three test samples are subjected to the above testing with the results being averaged and normalized to produce the reported adhesive bond strength.

### Elongation

The elongation of an elastic composite laminate according to the present invention is suitably determined as follows. A 2.54 cm (1-inch) wide by 10.16 cm (4-inch) long sample of the laminate is provided. The central 7.62 cm (3-inch) area of the sample is marked. The test sample is then stretched to its maximum length, and the distance between the marks is measured and recorded as the "stretched to stop length. "The percent elongation is determined according to the following formula:

### {(stretched to stop length (in inches))-3}/3 x 100

If a 2.54 cm by 10.16 cm (1-inch by 4-inch) area is not available, the largest sample possible (but less than 2.54 cm by 10.16 cm (1-inch by 4-inches)) is used for testing with the method being adjusted accordingly.

### Tension Force

The tension force of an elastic composite laminate according to the present invention is determined on a test sample of the laminate having a width of 2.54 cm (1 inch) and a length of 7.62 cm (3 inches). A test apparatus having a fixed clamp and an adjustable clamp is provided. The adjustable clamp is equipped with a strain gauge commercially available from S. A. Mieier Co. under the trade designation Chatillon DFIS2 digital force gauge. The test apparatus can elongate the test sample to a given length. One longitudinal end of the test sample is clamped in the fixed clamp of the test apparatus with the opposite longitudinal end being clamped in the adjustable clamp fitted with the strain gauge. The test sample is elongated to 90 percent of its elongation (as determined by the test method set forth above). The tension force is read from the digital force gauge after 1 minute. At least three samples of the elasticized area are tested in this manner with the results being averaged and reported as grams force per inch width.

### EXAMPLE 1

In this example, an elastomeric composite containing mutually reinforced elastomeric adhesive film and strand elements was prepared in accordance with the method of the invention. An elastomeric adhesive composition containing 40 wt% Dexco VECTOR 4111 SIS block polymer, 15 wt% Dexco VECTOR 4411 SIS block polymer, 45 wt% ESCOREZ 5340 tackifier, and 0.5 wt% Ciba IRGANOX 1010 antioxidant was blended in a Sigma mixer. The elastic adhesive (Brookfield viscosity = 47,000 cps@385 degrees Fahrenheit) was extruded from a melt tank as a film on a primary chill roll at 48 grams per square meter (gsm) before stretching. The melt tank temperature was in the range of 193 to 204 °C (380-400 degrees Fahrenheit) and the chill roll temperature was 11 degrees Celsius. The primary chill roll speed was 1.2 m per min (mbm) (4 ft per min (fpm)) and speeds for second, third, and nip rolls were respectively 2.1, 5.2 and 9.8 mpm (7,17, and 32 fpm).

The elastic strands were extruded onto the air side of the film on the second chill roll and immediately adhered on the film thereby forming a single unit composite. The strands were extruded at a spacing of 12 strands per 2.54 cm (1 inch) from a filament die having orifice diameters of 0.076 cm (0.030 inches). The strand output for the 30.5 cm (12-inch) wide die was 30 grams per minute (gpm). The strands were extruded from a twin screw extruder using a dryblend having the following composition: 90 wt% Dexco VECTOR 4111, 10 wt% Dexco ESCOREZ 5340. The melt temperature profile of the extruder was 120°C/175°C/190°C and hose and die temperatures were respectively 200 and 179 °C (355 degrees Fahrenheit).

This composite containing the adhesive film and strands adhered to it was stretched as a unit (amounting to 8 times the initial length of the film) and then laminated between two layers of 17 gsm (0.5 osy) Prism spunbond at a nip pressure of 655 kPa (95 psi). Neither the elastic film nor the strand showed breaks and the strands were self-aligned during the lamination process. The laminate made in this way appeared soft and gentle and exhibited high tension. The tension as measured shortly after production was 420 grams for a 5.08 cm (2-inch) wide sample which was stretched to 100% elongation from an initial length of 12.7 cm (5 inches). No delamination was observed after aging at 54.4°C (130 degrees Fahrenheit) for 1 week. The laminate also retained excellent elastic properties as evidenced by beneficially low and similar hysteresis loss factor values before and after aging, namely 19% before and 23% after aging, as well as high stretch-to-stop values in the range of 400%. Hysteresis loss factor was measured by the percentage difference between first cycle extension and second cycle retraction at 50% elongation.

### EXAMPLE 2

In this example, a strand-reinforced laminate material (sample B) was made in accordance with the invention and the tension decay properties were compared to a control (sample A) having the same elastomeric adhesive film composition without the elastic strands. In each case, the film add-on before stretching to 800% was 80 gsm and the elastomeric adhesive film composition was a mixture of 35 wt% PICOLYTES115 and 65% KRATON G2760, to which 10% Hercules PICOLYTE S25 was added. More particularly, the elastic adhesive film underwent process elongation of between 500% and 800%, resulting in an output basis weight of between 70 and 120 grams per square meter (gsm) before stretching onto a chill roll having a temperature of 10 to 15 degrees Celsius, from a melt tank having a temperature of up to 204°C (400 degrees Fahrenheit).

The control spunbond/film/spunbond laminate sample (sample A) was made without any reinforcing strands. The test sample (sample B) was reinforced with VFL extruded strands of tackified SBL styrenic block copolymer available under the trade designation KRATON@ G 2760 from Kraton Polymers, adhered to the elastic adhesive film. The test sample was prepared by extruding the strands and the elastomeric adhesive film on separate chill rolls. The die configuration through which the strands were extruded had 12 holes per 2.54 cm (1 inch) with a 0.076 cm (0.030 inch) diameter opening. The output of the strands in terms of grams per minute (gpm) was 29.5 gpm for 120 strands per 25.4 cm (10-inch) die width, extruded on a chill roll at a speed of 1.8 to 2.4 m (6-8 feet) per minute. Following the chill rolls, the strands and film were independently stretched to 700%. Thereafter, the film and strands were combined with spunbond webs on each side and laminated continuously to produce a material with tunable elastic properties.

Key process conditions versus physical properties (tension decay and adhesion) are summarized in Table 1. The tension decay was measured by first measuring the "green" tension at 100% elongation of a 10.16 cm (2-inch) wide, 12.7 cm (5-inch) long sample containing 24 strands (no strands in the case of the control sample). The tension reading was recorded from an electronic gauge one minute after clamping. After aging the samples at 54°C (130 degrees Fahrenheit) for 1 day, the "aged" tension was then measured in the same manner as the green tension and the resulting aged tension was compared to the green tension to determine whether, or to what extent, tension decay occurred. The tension decay value is calculated by the percent difference between green and aged tension readings. A lower value of tension decay is indicative of improved elastic material performance. The tension decay of the strand-reinforced composite is much lower (12.8%) than in the case of no strands (62%). In either case the formulation has excellent adhesion properties with no indications of delamination after continued aging at 54° C (130 degrees Fahrenheit) for 2 weeks.

By exhibiting no delamination, it is meant that the laminates cannot be peeled apart without facing material failure. In this Example, delamination was visually determined as opposed to carrying out a peel test. Visual observations focused on the presence of any air pockets detected between layers of the laminate.

Tension of the elastic composite is tunable by varying the strand add-on and stretch percentage, with greater add-on and greater stretch both resulting in greater tension.

Sample observations further reveal that soft feel and aesthetic properties of the laminates are preserved with the addition of the strands.

**Table 1: Comparison of Tension Decay and Adhesion Properties of Novel Elastic Composite Laminates**

| Sample | Output of extruded strands (gpm) | Strand process stretch (%) | Green tension (grams), 2-inch wide sample | Aged tension (grams), 1 day at 130° F | Tension decay (%) | Aged sample observations |
|---|---|---|---|---|---|---|
| A (control) | None | --- | 340 | 130 | 62.0 | No delamination |
| B (stranded composite) | 29.5 | 700 | 350 | 305 | 12.8 | No delamination |

### EXAMPLE 3

In this example, the strand-reinforced laminate material (sample B) of Example 2 was incorporated into the leg cuff portion of a personal care garment, as indicated by the reference number 30 in Fig. 8. The "in-product" tension properties were evaluated and compared to conventional spunbonded laminates (SBL) and Lycra laminates (including Lycra strands adhered with elastic attachment adhesive). The "in-product" tension values of the elasticized laminate composite portion of the personal care garment are shown in Table 2 and were measured as follows.

The garment was extended flat on a lightbox and clamped at one end while a 1000 gram weight in the form of a bar was attached at the other end, resulting in stretching of the material in the machine direction. Two marks corresponding to a discrete gauge length value in the range of 4-7 inches were placed on the stretched elastic portion. The weight was removed and the retracted elastic laminate composite was cut out from the product, normally as a 1-inch wide strip. The cut elastic strip was mounted in a Chantillon tension gauge and stretched to 90% of gauge length. The tension in grams was recorded after a one-minute waiting period. The elasticized strips were evaluated for tension before and after the product was worn at body temperature in order to evaluate the degree of tension decay as a result of wearing the article. A lower degree of tension decay (as measured by the percentage difference between post-wear and pre-wear) is indicative of a better performing elastic material. Tension measurements before and after wear show a significant drop for conventional SBL laminate control (-22%). The tension drop for the other control of conventional Lycra laminate was significant as well (-10%). However, the laminate made from the strand-reinforced elastomeric adhesive film of the invention had no tension loss and actually gained some tension as a result of wearing at body temperature. The elastic adhesive laminate of the invention with no tension loss provides superior gasketing performance compared to conventional materials.

**Table 2: Comparative Tension of Laminates**

| Material | Tension (grams), before wear | Tension (grams), after wear | % difference |
|---|---|---|---|
| SBL laminate | 127.5 | 104.5 | -22.0 |
| Lycra laminate | 120.8 | 109.4 | -10.0 |
| Strand-reinforced laminate | 141.5 | 144.6 | +2.1 |

It will be appreciated that details of the foregoing embodiments, given for purposes of illustration, are not to be construed as limiting the scope of this invention. Although only a few exemplary embodiments of this invention have been described in detail above, those skilled in the art will readily appreciate that many modifications are possible in the exemplary embodiments without materially departing from the novel teachings and advantages of this invention. Accordingly, all such modifications are intended to be included within the scope of this invention, which is defined in the following claims and all equivalents thereto. Further, it is recognized that many embodiments may be conceived that do not achieve all of the advantages of some embodiments, particularly of the preferred embodiments, yet the absence of a particular advantage shall not be construed to necessarily mean that such an embodiment is outside the scope of the present invention.

## Claims

1. An elastomeric composite laminate, comprising:
a plurality of extruded elastic strands adhered to an elastomeric adhesive film; and
a first facing sheet bonded to a first surface of the elastomeric adhesive film and a second facing sheet bonded to a second surface of the elastomeric adhesive film,
wherein the plurality of elastic strands comprises between 5% and 50% by weight of the film and the elastic strands combined, preferably between 10% and 35% by weight of the film and the elastic strands combined, preferably between 15% and 25% by weight of the film and the elastic strands combined.

2. The elastomeric composite laminate of Claim 1, wherein the plurality of extruded elastic strands is at least partially embedded in the elastomeric adhesive film.

3. The elastomeric composite laminate of Claim 1 or 2, wherein the plurality of elastic strands comprises at least one of the group consisting of raw polymers, a mixture of polymers, and tackified polymers.

4. The elastomeric composite laminate of Claim 1, 2 or 3, wherein the plurality of elastic strands comprises at least one of the group consisting of elastomeric polymer compositions, tackified polymers, olefin copolymers, ethylenepropylene-diene monomer, styrene-isoprene-styrene, styrene-butadiene-styrene, styrene-ethylene/butylene-styrene, styrene-ethylene/propylene-styrene, polyurethane, polyisoprene, cross-linked polybutadiene, and combinations thereof.

5. The elastomeric composite laminate of any preceding claim, wherein the plurality of elastic strands comprises a tackifier including at least one type of hydrocarbon selected from the group consisting of petroleum distillates, rosin, rosin esters, polyterpenes derived from wood, polyterpenes derived from synthetic chemicals, and combinations thereof.

6. The elastomeric composite laminate of any preceding claim, wherein the elastomeric adhesive film comprises an elastomeric, hot melt, pressure-sensitive adhesive.

7. The elastomeric composite laminate of any preceding claim, wherein at least one of the first and second facing sheets comprises a nonwoven web.

8. The elastomeric composite laminate of any preceding claim, wherein at least one of the first and second facing sheets comprises a film.

9. A garment having at least one opening for a body part, the garment comprising:
a substrate defining the at least one opening; and
an elastomeric composite attaching a facing sheet to the substrate adjacent the at least one opening, the elastomeric composite including a plurality of extruded elastic strands adhered to an elastomeric adhesive film wherein the plurality of elastic strands comprises between 5% and 50% by weight of the film and the elastic strands combined, preferably between 10% and 35% by weight of the film and the elastic strands combined, preferably between 15% and 25% by weight of the film and the elastic strands combined..

10. The garment of Claim 9, wherein the garment is one selected from the group consisting of personal care garments, medical garments, and industrial workwear garments, diapers, training pants, swim wear, absorbent underpants, adult incontinence products, feminine hygiene products, protective medical gowns, surgical medical gowns, caps, gloves, drapes, face masks, laboratory coats and coveralls.

11. The garment of Claim 9 or 10, wherein the at least one opening comprises a neck opening, a wrist opening, an arm opening, a waist opening, a leg opening, or an ankle opening.

12. The garment of Claim 9, 10 or 11, wherein the substrate comprises a leg cuff, or a containment flap.

13. A pant-like garment comprising:
a chassis including a substrate and defining a waist opening and two leg openings; and
an elastomeric composite attaching a facing sheet to the substrate adjacent each of the leg openings, the elastomeric composite including a plurality of extruded elastic strands adhered to an elastomeric adhesive film, wherein the plurality of elastic strands comprises between 5% and 50% by weight of the film and the elastic strands combined, preferably between 10% and 35% by weight of the film and the elastic strands combined, preferably between 15% and 25% by weight of the film and the elastic strands combined.

14. A method of making an elastomeric composite, comprising:
casting an elastomeric adhesive film onto a first chill roll;
separately extruding a plurality of elastic strands onto the film to create a strand-reinforced composite wherein the plurality of elastic strands comprises between 5% and 50% by weight of the film and the elastic strands combined, preferably between 10% and 35% by weight of the film and the elastic strands combined, preferably between 15% and 25% by weight of the film and the elastic strands combined; and
stretching the composite.

15. The method of Claim 14, further comprising passing the film onto a second chill roll and extruding the plurality of elastic strands onto the film while the film is in contact with the second chill roll.

16. The method of Claim 14 or 15, comprising stretching the composite by at least 200%.

17. The method of any of Claims 14 to 16, wherein the plurality of elastic strands is extruded onto the elastomeric adhesive film at an add-on rate of between 5 and 50 grams per square meter before stretching.

18. The method of Claim 17, further comprising controlling tension in the elastomeric composite through at least one of: selection of film composition, selection of strand composition, add-on rate, stretching the composite, selection of substrate, and combinations thereof.

19. The method of any of Claims 14 to 18, comprising casting the elastomeric adhesive film from a melt tank onto the first chill roll.

20. The method of any of Claims 14 to 18 comprising casting the elastomeric adhesive film from a supply roll onto the first chill roll.

21. The method of any of Claims 14 to 20, comprising extruding the plurality of elastic strands from a melt tank onto the film.

22. The method of any of Claims 14 to 20, comprising extruding the plurality of elastic strands from an extruder onto the film.

23. The method of any of Claims 14 to 20, comprising extruding the plurality of elastic strands from a supply roll onto the film.

24. A method of making an elastomeric composite laminate, comprising:
casting an elastomeric adhesive film onto a first chill roll;
passing the elastomeric adhesive film onto a second roll;
extruding a plurality of elastic strands onto the film while the film is on the second roll to create a mutually-reinforced strand/film composite, wherein the plurality of elastic strands comprises between 5% and 50% by weight of the film and the elastic strands combined, preferably between 10% and 35% by weight of the film and the elastic strands combined, preferably between 15% and 25% by weight of the film and the elastic strands combined;
stretching the composite;
laminating the stretched composite between a first facing sheet and a second facing sheet; and
controlling tension in the elastomeric composite through at least one of:
selection of film composition, selection of strand composition, add-on rate, stretching the composite, and combinations thereof.

25. A garment or method as claimed in any of Claims 13 to 24 in which the elastomeric composite is in the form of or forms part of a laminate as claimed in any of Claims 1 to 12.

## Patentansprüche

1. Elastomeres Verbundlaminat, umfassend:
eine Vielzahl extrudierter, elastischer Stränge, die an einer elastomeren Haftmittelfolie befestigt sind; und
ein erstes Deckblatt, das an eine erste Oberfläche der elastomeren Haftmittelfolie gebunden ist, und ein zweites Deckblatt, das an eine zweite Oberfläche der elastomeren Haftmittelfolie gebunden ist,
wobei die Vielzahl der elastischen Stränge zwischen 5 Gew.-% und 50 Gew.-% einer Kombination aus der Folie und den elastischen Strängen, bevorzugt zwischen 10 Gew.-% und 35 Gew.-% einer Kombination aus der Folie und den elastischen Strängen, bevorzugt zwischen 15 Gew.-% und 25 Gew.-% einer Kombination aus der Folie und den elastischen Strängen, umfasst.

2. Elastomeres Verbundlaminat nach Anspruch 1, worin die Vielzahl der extrudierten elastischen Stränge zumindest teilweise in die elastomere Haftmittelfolie eingebettet ist.

3. Elastomeres Verbundlaminat nach Anspruch 1 oder 2, wobei die Vielzahl der elastischen Stränge mindestens einen aus der Gruppe, die aus Rohpolymeren, einem Gemisch aus Polymeren und klebriggemachten Polymeren besteht, umfasst.

4. Elastomeres Verbundlaminat nach Anspruch 1, 2 oder 3, wobei die Vielzahl der elastischen Stränge mindestens einen aus der Gruppe, die aus elastomeren Polymerzusammensetzungen, klebriggemachten Polymeren, Olefin-Copolymeren, Ethylen-Propylen-Dien-Monomer, Styren-Isopren-Styren, Styren-Butadien-Styren, Styren-Ethylen/Butylen-Styren, Styren-Ethylen/Propylen-Styren, Polyurethan, Polyisopren, vernetztem Polybutadien, und Kombinationen daraus besteht, umfasst.

5. Elastomeres Verbundlaminat nach einem der vorhergehenden Ansprüche, wobei die Vielzahl an elastischen Strängen einen Klebrigmacher enthält, der mindestens eine Art von Kohlenwasserstoff einschließt, ausgewählt aus der Gruppe, die aus Erdöldestillaten, Kollophonium, Harzestern, von Holz abgeleiteten Polyterpenen, von synthetischen Chemikalien abgeleiteten Polyterpenen, und Kombinationen daraus besteht.

6. Elastomeres Verbundlaminat nach einem der vorhergehenden Ansprüche, wobei die elastomere Haftmittelfolie ein elastomeres, druckempfindliches Heiß-Schmelz-Haftmittel umfasst.

7. Elastomeres Verbundlaminat nach einem der vorhergehenden Ansprüche, wobei mindestens eines der ersten oder zweiten Deckblätter eine Vliesstoffbahn umfasst.

8. Elastomeres Verbundlaminat nach einem der vorhergehenden Ansprüche, wobei mindestens eines der ersten oder zweiten Deckblätter eine Folie umfasst.

9. Bekleidungsstück, das mindestens eine Öffnung für einen Körperteil aufwesit, wobei das Bekleidungsstück umfasst:
ein Substrat, das die mindestens eine Öffnung definiert; und
ein elastomeres Verbundmaterial, das ein Deckblatt an dem Substrat angrenzend an die mindestens eine Öffnung befestigt, wobei das elastomere Verbundmaterial eine Vielzahl an extrudierten elastischen Strängen einschließt, die an einer elastomeren Haftmittelfolie befestigt sind, wobei die Vielzahl der elastischen Stränge zwischen 5 Gew.-% und 50 Gew.-% einer Kombination aus der Folie und den elastischen Strängen, bevorzugt zwischen 10 Gew.-% und 35 Gew.-% einer Kombination aus der Folie und den elastischen Strängen, bevorzugt zwischen 15 Gew.-% und 25 Gew.-% einer Kombination aus der Folie und den elastischen Strängen, umfasst.

10. Bekleidungsstück nach Anspruch 9, wobei das Bekleidungsstück aus der Gruppe ausgewählt ist, die aus Bekleidungsstücken zur Körperpflege, medizinischen Bekleidungsstücken und industriellen Arbeitsbekleidungsstücken, Windeln, Übungshöschen, Badebekleidung, absorbierenden Unterhosen, Inkontinenz-Produkten für Erwachsene, Produkten für die weibliche Hygiene, medizinischen Schutzbekleidungsstücken, medizinischen OP-Bekleidungsstücken, Hauben, Handschuhen, Abdecktüchem, Gesichtsmasken, Labormänteln und Overalls besteht.

11. Bekleidungsstück nach Anspruch 9 oder 10, wobei die mindestens eine Öffnung eine Halsöffnung, eine Handgelenksöffnung, eine Armöffnung, eine Taillenöffnung, eine Beinöffnung oder eine Fußknöchelöffnung umfasst.

12. Bekleidungsstück nach Anspruch 9, 10 oder 11, wobei das Substrat eine Manschette an der Beinöffnung oder eine Auslaufschutzklappe umfasst.

13. Hosenähnliches Bekleidungsstück, umfassend:
ein Gehäuse, das ein Substrat einschließt und eine Taillenöffnung und zwei Beinöffnungen definiert; und
ein elastomeres Verbundmaterial, das ein Deckblatt an dem Substrat angrenzend an jede der Beinöffnungen befestigt, wobei das elastomere Verbundmaterial eine Vielzahl an extrudierten elastischen Strängen einschließt, die an einer elastomeren Haftmittelfolie befestigt sind, wobei die Vielzahl der elastischen Stränge zwischen 5 Gew.-% und 50 Gew.-% einer Kombination aus der Folie und den elastischen Strängen, bevorzugt zwischen 10 Gew.-% und 35 Gew.-% einer Kombination aus der Folie und den elastischen Strängen, bevorzugt zwischen 15 Gew.-% und 25 Gew.-% einer Kombination aus der Folie und den elastischen Strängen, umfasst.

14. Verfahren zur Herstellung eines elastomeren Verbundmaterials, umfassend:
Gießen einer elastomeren Haftmittelfolie auf eine erste Kühlwalze;
separates Extrudieren einer Vielzahl von elastischen Strängen auf die Folie, um ein strangverstärktes Verbundmaterial zu bilden, wobei die Vielzahl der elastischen Stränge zwischen 5 Gew.-% und 50 Gew.-% einer Kombination aus der Folie und den elastischen Strängen, bevorzugt zwischen 10 Gew.-% und 35 Gew.-% einer Kombination aus der Folie und den elastischen Strängen, bevorzugt zwischen 15 Gew.-% und 25 Gew.-% einer Kombination aus der Folie und den elastischen Strängen, umfasst; und
Strecken des Verbundmaterials.

15. Verfahren nach Anspruch 14, weiterhin umfassend das Leiten der Folie auf eine zweite Kühlwalze und Extrudieren der Vielzahl der elastischen Stränge auf die Folie während die Folie mit der zweiten Kühlwalze in Kontakt steht.

16. Verfahren nach Anspruch 14 oder 15, umfassend das Strecken des Verbundmaterials um mindestens 200%.

17. Verfahren nach einem der Ansprüche 14 bis 16, wobei die Vielzahl der elastischen Stränge auf die elastomere Haftmittelfolie mit einer Zugaberate zwischen 5 und 50 Gramm pro Quadratmeter vor dem Strecken extrudiert wird.

18. Verfahren nach Anspruch 17, weiterhin umfassend das Kontrollieren der Spannung in dem elastomeren Verbundmaterial durch mindestens eines aus: Auswahl der Folienzusammensetzung, Auswahl der Strangzusammensetzung, Zugaberate, Strecken des Verbundmaterials, Auswahl des Substrates, und Kombinationen daraus.

19. Verfahren nach einem der Ansprüche 14 bis 18, umfassend das Gießen der elastomeren Haftmittelfolie aus einem Schmelztank auf die erste Kühlwalze.

20. Verfahren nach einem der Ansprüche 14 bis 18, umfassend das Gießen der elastomeren Haftmittelfolie von einer Zuführwalze auf die erste Kühlwalze.

21. Verfahren nach einem der Ansprüche 14 bis 20, umfassend das Extrudieren der Vielzahl von elastischen Strängen aus einem Schmelztank auf die Folie.

22. Verfahren nach einem der Ansprüche 14 bis 20, umfassend das Extrudieren der Vielzahl von elastischen Strängen von einem Extruder auf die Folie.

23. Verfahren nach einem der Ansprüche 14 bis 20, umfassend das Extrudieren der Vielzahl von elastischen Strängen von einer Zuführwalze auf die Folie.

24. Verfahren zur Herstellung eines elastomeren Verbundlaminats, umfassend:
Gießen einer elastomeren Haftmittelfolie auf eine erste Kühlwalze;
Leiten der elastomeren Haftmittelfolie auf eine zweite Walze;
Extrudieren einer Vielzahl von elastischen Strängen auf die Folie während sich die Folie auf der zweiten Walze befindet, um ein gegenseitig verstärktes Strang/Folien-Verbundmaterial zu schaffen, wobei die Vielzahl der elastischen Stränge zwischen 5 Gew.-% und 50 Gew.-% einer Kombination aus der Folie und den elastischen Strängen, bevorzugt zwischen 10 Gew.-% und 35 Gew.-% einer Kombination aus der Folie und den elastischen Strängen, bevorzugt zwischen 15 Gew.-% und 25 Gew.-% einer Kombination aus der Folie und den elastischen Strängen, umfasst;
Strecken des Verbundmaterials;
Laminieren des gestreckten Verbundmaterials zwischen ein erstes Deckblatt und ein zweites Deckblatt; und
Kontrollieren der Spannung in dem elastomeren Verbundmaterial durch mindestens eines aus: Auswahl der Folienzusammensetzung, Auswahl der Strangzusammensetzung, Zugaberate, Strecken des Verbundmaterials, und Kombinationen daraus.

25. Bekleidungsstück oder Verfahren nach einem der Ansprüche 13 bis 24, in dem das elastomere Verbundmaterial in Form eines Laminats vorliegt oder einen Teil eines Laminats nach einem der Ansprüche 1 bis 12 bildet.

## Revendications

1. Stratifié composite élastomère, comprenant :
une pluralité de brins élastiques extrudés collés à un film adhésif élastomère ; et
une première feuille de façade liée à une première surface du film adhésif élastomère et une seconde feuille de façade liée à une seconde surface du film adhésif élastomère,
dans lequel les brins élastiques de la pluralité représentent entre 5 % et 50 % en poids du film et des brins élastiques combinés, de préférence entre 10 % et 35 % en poids du film et des brins élastiques combinés, de préférence entre 15 % et 25 % en poids du film et des brins élastiques combinés.

2. Stratifié composite élastomère selon la revendication 1, dans lequel les brins élastiques extrudés de la pluralité sont au moins partiellement noyés dans le film adhésif élastomère.

3. Stratifié composite élastomère selon la revendication 1 ou 2, dans lequel les brins élastiques de la pluralité comprennent au moins un membre du groupe consistant en les polymères bruts, un mélange de polymères et des polymères rendus pégueux.

4. Stratifié composite élastomère selon la revendication 1, 2 ou 3, dans lequel les brins élastiques de la pluralité comprennent au moins un membre du groupe consistant en les compositions polymères élastomères, les polymères rendus pégueux, les copolymères d'oléfines, le monomère éthylène-propylène-diène, le styrène-isoprène-styrène, le styrène-butadiène-styrène, le styrène-éthylène/butylène-styrène, le styrène-éthylène/ propylène-styrène, le polyuréthane, le polyisoprène, le polybutadiène réticulé, et leurs combinaisons.

5. Stratifié composite élastomère selon l'une quelconque des revendications précédentes, dans lequel la pluralité de brins élastiques comprend un agent de pégosité incluant au moins un type d'hydrocarbure sélectionné dans le groupe consistant en les distillats de pétrole, la colophane, les esters de colophane, les polyterpènes dérivant du bois, les polyterpènes dérivant de produits chimiques de synthèse, et leurs combinaisons.

6. Stratifié composite élastomère selon l'une quelconque des revendications précédentes, dans lequel le film adhésif élastomère comprend un adhésif de fusion, élastomère, sensible à la pression.

7. Stratifié composite élastomère selon l'une quelconque des revendications précédentes, dans lequel l'une au moins de la première feuille de façade et de la seconde feuille de façade comprend un voile non-tissé.

8. Stratifié composite élastomère selon l'une quelconque des revendications précédentes, dans lequel l'une au moins de la première feuille de façade et de la seconde feuille de façade comprend un film.

9. Vêtement ayant au moins une ouverture pour une partie corporelle, le vêtement comprenant :
un substrat définissant ladite au moins une ouverture ; et
un composite élastomère fixant une feuille de façade au substrat au voisinage de ladite au moins une ouverture, le composite élastomère incluant une pluralité de brins élastiques extrudés collés à un film adhésif élastomère, où les brins élastiques de la pluralité représentent entre 5 % et 50 % en poids du film et des brins élastiques combinés, de préférence entre 10 % et 35 % en poids du film et des brins élastiques combinés, de préférence entre 15 % et 25 % en poids du film et des brins élastiques combinés.

10. Vêtement selon la revendication 9, ledit vêtement étant sélectionné dans le groupe consistant en les vêtements d'hygiène personnelle, les vêtements médicaux et les vêtements de travail industriels, les couches-culottes, les culottes d'apprentissage de la propreté, les vêtements de natation, les sous-vêtements absorbants, les produits pour l'incontinence adulte, les produits d'hygiène féminine, les blouses médicales protectrices, les blouses médicales et chirurgicales, les bonnets, les gants, les champs opératoires, les masques faciaux, les blouses de laboratoire et les combinaisons.

11. Vêtement selon la revendication 9 ou 10, dans lequel ladite au moins une ouverture comprend une ouverture de col, une ouverture de poignet, une ouverture d'emmanchure, une ouverture de ceinture, une ouverture de jambe ou une ouverture de bas de jambe.

12. Vêtement selon la revendication 9, 10 ou 11, dans lequel le substrat comprend un tour de jambe ou un volet de confinement.

13. Vêtement semblable à une culotte, comprenant :
une structure incluant un substrat et définissant une ouverture de ceinture et deux ouvertures de jambe ; et
un composite élastomère fixant une feuille de façade au substrat au voisinage de chacune des ouvertures de jambe, le composite élastomère incluant une pluralité de brins élastiques extrudés collés à un film adhésif élastomère, où les brins élastiques de la pluralité représentent entre 5 % et 50 % en poids du film et des brins élastiques combinés, de préférence entre 10 % et 35 % en poids du film et des brins élastiques combinés, de préférence entre 15 % et 25 % en poids du film et des brins élastiques combinés.

14. Procédé de fabrication d'un composite élastomère, comprenant :
le coulage d'un film adhésif élastomère sur un premier rouleau de refroidissement ;
l'extrusion séparée d'une pluralité de brins élastiques sur le film pour créer un composite renforcé par des brins, dans lequel les brins élastiques de la pluralité représentent entre 5 % et 50 % en poids du film et des brins élastiques combinés, de préférence entre 10 % et 35 % en poids du film et des brins élastiques combinés, de préférence entre 15 % et 25 % en poids du film et des brins élastiques combinés ; et
l'étirage du composite.

15. Procédé selon la revendication 14, comprenant, en outre, le passage du film sur un second rouleau de refroidissement et l'extrusion de la pluralité de brins élastiques sur le film tandis que le film est en contact avec le second rouleau de refroidissement.

16. Procédé selon la revendication 14 ou 15, comprenant l'étirage du composite d'au moins 200 %.

17. Procédé selon l'une quelconque des revendications 14 à 16, dans lequel les brins élastiques de la pluralité sont extrudés sur le film adhésif élastomère à un taux d'adjonction compris entre 5 et 50 grammes par mètre carré avant l'étirage.

18. Procédé selon la revendication 17, comprenant, en outre, la maîtrise de la tension dans le composite élastomère par l'un au moins des moyens suivants : la sélection de la composition du film, la sélection de la composition des brins, le taux d'adjonction, l'étirage du composite, la sélection du substrat, et leurs combinaisons.

19. Procédé selon l'une quelconque des revendications 14 à 18, comprenant le coulage du film adhésif élastomère depuis un réservoir de produit en fusion sur le premier rouleau de refroidissement.

20. Procédé selon l'une quelconque des revendications 14 à 18, comprenant le coulage du film adhésif élastomère depuis un rouleau d'alimentation sur le premier rouleau de refroidissement.

21. Procédé selon l'une quelconque des revendications 14 à 20, comprenant l'extrusion de la pluralité de brins élastiques depuis un réservoir de produit en fusion sur le film.

22. Procédé selon l'une quelconque des revendications 14 à 20, comprenant l'extrusion de la pluralité de brins élastiques depuis une extrudeuse sur le film.

23. Procédé selon l'une quelconque des revendications 14 à 20, comprenant l'extrusion de la pluralité de brins élastiques depuis un rouleau d'alimentation sur le film.

24. Procédé de fabrication d'un stratifié composite élastomère, comprenant :
le coulage d'un film adhésif élastomère sur un premier rouleau de refroidissement ;
le passage du film adhésif élastomère sur un second rouleau ;
l'extrusion d'une pluralité de brins élastiques sur le film tandis que le film est sur le second rouleau pour créer un composite brins/film dont les composants se renforcent mutuellement, composite dans lequel les brins élastiques de la pluralité représentent entre 5 % et 50 % en poids du film et des brins élastiques combinés, de préférence entre 10 % et 35 % en poids du film et des brins élastiques combinés, de préférence entre 15 % et 25 % en poids du film et des brins élastiques combinés ;
l'étirage du composite ;
la stratification du composite étiré entre une première feuille de façade et une seconde feuille de façade ; et
la maîtrise de la tension dans le composite élastomère par l'un au moins des moyens suivants : la sélection de la composition du film, la sélection de la composition des brins, le taux d'adjonction, l'étirage du composite, la sélection du substrat, et leurs combinaisons.

25. Vêtement ou procédé selon l'une quelconque des revendications 13 à 24, dans lequel le composite élastomère est sous la forme, ou fait partie, d'un stratifié selon l'une quelconque des revendications 1 à 12.
